# EUROPEAN PATENT SPECIFICATION

(11) **EP 4 520 246 B1**
(45) Date of publication and mention of the grant of the patent: **28.10.2026**
(21) Application number: 23195491.8
(22) Date of filing: 05.09.2023
(51) Int. Cl.: A61B 1/00, A61B 1/015

(54) **ENDOSCOPE COMPRISING A SUCTION VALVE**
ENDOSKOP MIT SAUGVENTIL
ENDOSCOPE COMPRENANT UNE SOUPAPE D'ASPIRATION

(43) Date of publication of application: 12.03.2025
(73) Proprietor: Ambu A/S, 2750 Ballerup (DK)
(72) Inventor: MORTON, Alistair David, 2300 Copenhagen (DK)
(74) Representative: Winter, Brandl - Partnerschaft mbB

(56) References cited:
- EP-A1- 3 854 291
- US-A- 5 322 263
- US-A1- 2023 157 523
- US-A1- 2023 200 632

## Description

The present disclosure relates to an endoscope comprising a proximal endoscope handle or interface and an insertion cord extending from the endoscope handle or interface and configured to be inserted into a patient's body cavity, the endoscope having a suction valve mounted in the endoscope handle or interface. The present disclosure further relates to a method of assembling the endoscope and a system comprising an endoscope and a video processing apparatus (VPA).

### Related art

Endoscopes and similar specialized instruments such as bronchoscopes, arthroscopes, colonoscopes, laparoscopes, gastroscopes, and duodenoscopes are well-known from the related art and are typically used for visual examination and diagnosis of hollow organs and body cavities, as well as to assist in surgery, e.g. for a targeted tissue sampling. Both reusable and disposable endoscopes are known from the related art. Known endoscopes usually comprise: an endoscope handle or interface; an insertion cord extending from the endoscope handle or interface, configured to be inserted into a patient's body cavity and comprising an insertion tube, a bending section and a distal tip unit; and a working channel. The working channel extends from the working channel access port at the endoscope handle to the distal tip unit.

When examining an object such as a body cavity or hollow organ with an endoscope it is desirable to have a clear and good view or visibility of the examined object. However, the visibility of such an object is often affected by undesirable fluid or mucus, which may obstruct visibility of the object to be examined. Furthermore, it may be desirable to remove tissue parts or particles during the surgery for example to extract a sample for further analysis, or to provide better visibility. It is thus desirable to remove such undesirable fluid or mucus, as well as such tissue parts or particles, using a suction device, such as a vacuum pump.

It is desirable that the surgeon can start and interrupt the suction at any time. Basically, it would be possible to control a vacuum pump performance for this purpose. However, this is associated with considerable delays caused by a startup and a shutdown of the pump motor.

In order to overcome these disadvantages, suction valves have become established in endoscopes, with which the effect of the suction power constantly applied by the vacuum pump can be applied or switched off. Further general requirements for such a suction valve may be that the suction valve is easy and quick to operate, and closes reliably and safely.

Various suction valves for endoscopes are known in the related art.

Basically, suction valves comprising a housing and a piston that is movable within the housing are well-known.

For example, EP 3 854 291 A1 discloses a suction valve assembly including a housing and a plunger, which moves up and downwards to open or close the suction valve.

US 2023/200632 A1 discloses a suction valve body with a suction valve button, which is used to control suction.

US 5 871 441 A discloses a suction valve that can be connected to a working channel of an endoscope and includes a preferably cylindrical housing, a piston, a spring and a button. The piston is provided with a flow channel and is movably accommodated within the housing. The button preferably on top of the piston is connected to the piston such that the piston is movable together with the button. An operator can press the button to move the piston from a valve closed state to a valve open state. The spring ensures that the valve is usually in the valve closed state, in particular when the operator does not press down the button. The housing is provided with an inlet opening that is connected to the working channel of the endoscope and with an outlet opening that is connected to a suction device. Usually, the suction device is active or running. When the valve is in the valve closed state an outer circumferential surface of the cylindrically shaped piston blocks the inlet opening. When the operator presses down the button, the piston is moved downwards such that the flow channel of the piston connects the inlet opening of the housing with the outlet opening of the housing, and a fluid flow through the suction valve is enabled. Hence, fluid or mucus or tissue parts orparticles can be sucked from the working channel of the endoscope through the suction valve.

Suction valves known from the related art are usually securely and sealingly positioned and fixed in the housing of the endoscope handle to ensure a proper functioning of the endoscope.

In related art endoscopes and especially single-use endoscopes, the suction valve is usually glued into the housing of the handle. Such gluing involves considerable assembly work, which makes the endoscope more complex to manufacture and thus more expensive.

US 5 299 561 A discloses a configuration in which the suction valve is formed integrally with the housing and a valve spring is to be inserted or mounted into the monolithic housing. Such a monolithic design has the disadvantage that the complex geometry of the valve has to be formed directly in the housing, which significantly increases the complexity of the housing and thus the manufacturing costs.

### Brief description of the disclosure

The object of the present disclosure is therefore to overcome or at least reduce the disadvantages of the related art. More specifically, it is the purpose of the present disclosure to provide an endoscope having a suction valve, wherein the endoscope is formed from components that are simple and inexpensive to manufacture, and wherein the endoscope is easy to assemble.

This object is solved by an endoscope according to independent claim 1. The object is further solved by a method according to independent claim 11 and by a system according to claim 12.

In detail, according to the invention as defined in claim 1, the endoscope comprises a proximal endoscope handle or interface, an insertion cord extending from the endoscope handle or interface and configured to be inserted into a patient's body cavity and a working channel configured to let fluid and mucus sucked from the patient's body cavity flow therethrough. The endoscope handle or interface comprises a housing and a suction valve configured to control a suction through the working channel and having a valve closed state and a valve open state. The housing comprises a first half-shell and a second half-shell The suction valve comprises a valve body having an inlet connected to the working channel, an outlet provided and configured to be connected to a suction device and a stem movable axially in the valve body between at least a first position, in which the suction valve is in the valve closed state, and a second position, in which the suction valve is in the valve open state. The suction valve is mounted between the first half-shell and the second half-shell. The first half-shell comprises a first, outer fixing portion and a second, inner fixing portion. The first, outer fixing portion is configured to receive a first portion of the suction valve, which is a valve spring, and the second, inner fixing portion is configured to receive a second portion of the suction valve. The second half-shell comprises a third, outer fixing portion and a fourth, inner fixing portion, wherein the third, outer fixing portion is configured to receive the first portion of the suction valve, and wherein the fourth, inner fixing portion is configured to receive the second portion of the suction valve. The valve spring is formed by an elastic material, and the first outer fixing portion of the first half-shell and the third outer fixing portion of the second half-shell contact and clamp the valve spring of the suction valve in an assembled state of the first and second half-shells.

In other words, the present disclosure relates to an endoscope, preferably a single use endoscope, comprising the endoscope handle or interface. In other words, the handle may be substituted by the interface. Alternatively, it is conceivable that the interface is formed on the handle. The handle may contain essential control elements, such as a lever for controlling bending of the endoscope for the endoscope and ports for accessories such as a video processing apparatus or the suction device, for example in the form of a vacuum pump. The handle is preferably designed in such a way that it can be ergonomically gripped and operated by the user or operator or surgeon with just one hand. This means that all important operating elements on the handle are preferably arranged in such a way that the user can operate them with one hand without having to change a position of his or her hand. The handle comprises a shell. Preferably, the shell is made from a plastic material. Furthermore, the shell comprises two half-shells, a first half-shell and a second half-shell which are connectable with each other in a separation plane or dividing plane or split plane. In case of a provision of a proximal endoscope interface, said endoscope interface may be prepared or provided to be connectable to e.g. a robotic arm.

The endoscope further comprises the insertion cord, preferably having a distal tip unit formed at the distal end of the insertion cord. The distal tip unit may comprise an image capturing means like a camera and a light emitting device like a LED.

Furthermore, the endoscope comprises the working channel. The working channel is provided and configured to transport fluid, mucus and tissue parts or particles from the patient's body cavity. Furthermore, the working channel may be configured to guide a surgical tool or instrument through it to a surgical location in the patient's body. For this purpose, the working channel may have a Y-connector in the handle that allows insertion of the surgical tool or instrument into the working channel.

The endoscope further comprises the suction valve arranged in the endoscope handle or interface.

The suction valve comprises the valve body, which may also be designated as a valve housing. The valve body may be formed cylindrically at least in portions and may thus be described as including a valve cylinder that extends in an axial direction of the suction valve.

The inlet and the outlet of the suction valve are preferably formed as integral parts or portions of the valve body. The inlet and the outlet may have substantially a geometry of a pipe section, preferably of a pipe section with an annular cross-section.

The inlet and outlet may extend away from an outer cylindrical surface of the valve cylinder of the valve body.

The inlet is provided and configured to be connected to the working channel. The inlet may be connected directly to the working channel. Alternatively, the inlet may be connected to the working channel via a suction channel, especially preferably to the Y-connector of the working channel. The suction channel may e.g. be configured as a separate hose or tube.

The outlet is provided and configured to be connected to the suction device. The outlet may be connected directly to the suction device. Alternatively, a hose may be formed between the outlet and the suction device. Alternatively or additionally, at least one coupling element and/or at least one deflector element and/or at least one pipe element may be formed between the outlet and the suction device.

The suction valve further comprises the stem. The stem is arranged in the valve body in such a way that the stem is able to move in the axial direction of the suction valve in the valve body, especially in the valve cylinder of the valve body. The axial movement, at least in one direction, can be caused by pushing a button. The button may be a button portion which is preferably formed integrally with preferably a remainder of the stem at an end portion of the stem. Alternatively, the button may be a button element attached to the stem, in particular to the end portion of the stem. The button portion or element may protrude axially from the valve body, so that a user can press the button portion or element in order to move the stem axially in the valve body. The stem can be moved in the valve body in such a way that it shifts the suction valve at least between the valve closed state of the suction valve, in other words in its first position, and the valve open state of the suction valve, in other words in its second position. In other words, the stem may be configured to seal between an inlet opening and an outlet opening in the suction valve closed state, and the stem may be configured to enable a fluid flow between the inlet opening and the outlet opening of the valve body in the suction valve open state. In still other words, the suction valve may be configured to manually control the suction through the working channel, in particular by the user pressing the button towards the housing of the endoscope. In this way, the user can easily control the suction valve and can switch between the valve closed state and the valve open state.

The suction valve is mounted between the first half-shell and the second half-shell. In other words, the suction valve is arranged in the dividing plane of the handle and is fixed to the first half-shell and/ or the second half-shell.

The suction valve may be locked in the first half-shell and/ or the second half-shell.

The first half-shell comprises the first fixing portion and the second fixing portion. The first fixing portion is positioned further outwardly than the second fixing portion. In other words, the first fixing portion is positioned closer to a circumferential or peripheral edge of the shell, which is positioned in the dividing plane, than the second fixing portion.

The first fixing portion is provided and configured to receive the first portion of the suction valve, which is the valve spring. "Receiving" means that the first portion is held by the first fixing portion, for example in a form fitting manner. Alternatively or additionally, a frictional connection such as press fit may be provided.

The second fixing portion is provided and configured to receive the second portion of the suction valve. "Receiving" means that the second portion is held by the second fixing portion, for example in a form fitting manner. Alternatively or additionally, a frictional connection may be provided.

The first portion of the suction valve is different from the second portion of the suction valve. In other words, the first portion is spaced from the second portion.

Preferably, the first portion and the second portion of the suction valve may be formed of different materials.

Further preferably, the first portion and the second portion may have substantially different geometric shapes. For example, the first portion may have a round geometry and the second portion may have an angular geometry.

Such a configuration of the endoscope allows the suction valve to be positioned and fixed in the handle easily and without the use of tools. The formation of two different mounting points between the suction valve and the first half-shell ensures that the suction valve is firmly fixed in the handle without twisting. Furthermore, such a configuration can prevent incorrect assembly, for example by the poka yoke principle.

The suction valve, in particular the parts thereof, may be made of a plastic material or polymer. Especially preferred, the suction valve, in particular parts thereof, preferably the valve body and the stem, may be manufactured in an injection molding process, i.e. may be injection molded parts.

The second half-shell comprises a third, outer fixing portion and a fourth, inner fixing portion. The third, outer fixing portion is configured to receive the first portion of the suction valve, and the fourth, inner fixing portion is configured to receive the second portion of the suction valve.

The third fixing portion is provided and configured to receive the first portion of the suction valve, which is the valve spring. "Receiving" means that the first portion is held by the third fixing portion, for example in a form fitting manner. Alternatively or additionally, a frictional connection may be provided.

The fourth fixing portion is provided and configured to receive the second portion of the suction valve. "Receiving" means that the second portion is held by the fourth fixing portion, for example in a form fitting manner. Alternatively or additionally, a frictional connection may be provided.

Since the first half-shell and the second half-shell are mounted to each other to form the handle, it is advantageous if the third fixing portion and the fourth fixing portion, which also secure the suction valve, are formed on the second half-shell. In this way, the mounting of the suction valve can be further improved and a more rigid connection between the first half-shell, the second half-shell and the suction valve can be achieved.

In another aspect, the first outer fixing portion of the first half-shell and the third outer fixing portion of the second half-shell may be formed identically or symmetrically to each other.

In other words, the third fixing portion of the second half-shell may correspond to the first fixing portion of the first half-shell, and/ or the third fixing portion of the second half-shell may correspond to the first fixing portion of the first half-shell mirrored on the split plane or dividing plane of the handle.

Such a matched configuration of the first fixing portion and the third fixing portion ensures uniform force transmission between the suction valve, the first half-shell and the second half-shell. This is particularly advantageous because the first fixing portion and the third fixing portion are each the outer fixing portions, which are close to a force application point of the suction valve.

In another aspect, the first, outer fixing portion of the first half-shell and the third, outer fixing portion of the second half-shell may preferably substantially completely encircle the first portion of the suction valve in a circumferential direction.

In other words, the first fixing portion and the third fixing portion may each have a semi-circular shape which, in the assembled state, that is, in the state when the first half-shell is assembled to the second half-shell and thus the housing or handle shell is formed, form a circular shape which completely encloses the first portion of the suction valve, which may be circular in shape.

In still other words, the first fixing portion and the third fixing portion may form a collar shape that encloses the first portion of the suction valve, which is the valve spring.

Such a configuration of the first fixing portion and the third fixing portion can, in addition to further improving the force transmission, implement a seal between the suction valve and the handle. In particular, the sealing can be implemented without additional sealing elements, which further reduces the cost of the endoscope and makes the endoscope easier and cheaper to assemble.

In another aspect, the first fixing portion and/ or the third fixing portion may be formed as a portion of the handle shell, preferably of the half shell, having a groove or a recess.

In another aspect, the second, inner fixing portion of the first half-shell and the fourth inner fixing portion of the second half-shell may be formed differently.

In other words, the second fixing portion and the fourth fixing portion may have different geometries.

Preferably, the mounting in the second fixing portion may differ from the mounting in the fourth fixing portion. Alternatively or additionally, a clearance of the second fixing portion may differ from a clearance of the fourth fixing portion.

Different fixations in the second fixing portion and the fourth fixing portion can facilitate an assembly process of the handle. Furthermore, excessive tensioning of the suction valve between the second fixing portion of the first half-shell and the fourth fixing portion of the second half-shell can be prevented by the different fixations.

In another aspect, the second, inner fixing portion of the first half-shell and the fourth inner fixing portion of the second half-shell may be configured as tower geometries protruding from outer shell portions or shell skins of the half-shells into an interior space of the endoscope handle or interface.

In another aspect, the second, inner fixing portion of the first half-shell may comprise a slot of constant width.

In other words, the second fixing portion may be slot-shaped.

The slot of constant width may preferably be formed on a first tower geometry. A first tower geometry is understood to be a geometry, which extends preferably perpendicularly away from an outer shell portion of the first half-shell in the direction of the interior of the handle. Preferably, the extension towards the interior is greater than an extension parallel to the outer shell portion/ shell skin.

In another aspect, the fourth inner fixing portion of the second half-shell may comprise a V-shaped slot.

In other words, the fourth fixing portion may be V-shaped.

The V-shaped slot may preferably be formed on a second tower geometry. A second tower geometry is understood to be a geometry, which extends substantially perpendicularly away from an outer shell portion or shell skin of the second half-shell in the direction of the interior of the handle. Preferably, the extension towards the interior is greater than an extension parallel to the outer shell portion or shell skin.

Such a configuration of the second fixing portion and the fourth fixing portion can further simplify the assembly of the handle. The second, slot-shaped fixing portion in the first half-shell can firmly position the suction valve, and the V-shape of the fourth fixing portion can significantly simplify the process of introducing the portion of the suction valve into the fourth fixing portion, which is particularly advantageous when assembling the first half-shell with the second half-shell, where a view of the fourth fixing portion is obscured by the second half-shell.

The tower geometries make it possible to hold the suction valve in the center of the handle without having to make the corresponding portion on the suction valve too long, which could reduce the stability of the portion.

The first portion of the suction valve is the valve spring.

In other words, the first fixing portion of the first half-shell and the third fixing portion of the second half-shell contact and hold the valve spring of the suction valve, preferably circumferentially.

The valve spring is formed by an elastic material, in particular an elastomer.

By fixing the suction valve via the valve spring, a reliable sealing between the handle and the suction valve can be achieved. This sealing is further increased because the valve spring is made of an elastic material, in particular an elastomer. In addition, the formation of further fastening geometries on the suction valve can be omitted, which reduces the number of components or the complexity of the components and thus the costs and assembly effort for the suction valve and the endoscope.

In another aspect, the second portion of the suction valve may be at least one projection formed integrally with the valve body.

In other words, the second portion may be an integral section of the valve body, which is injection molded together with the valve body. The second portion may include at least one projection, preferably two projections.

In another aspect, the second portion may comprise a first projection formed integrally with the valve body and a second projection formed integrally with the valve body, said first projection and said second projection may project in opposite directions from said valve body, in particular from the valve cylinder of the valve body.

In other words, the second portion may comprise the two wing-shaped projections formed radially opposite each other on the valve body. The first projection and the second projection may be symmetrical or identical. Alternatively, it is conceivable to form the first projection and the second projection differently, for example to implement the poka yoke principle. The first projection and the second projection may differ in width, length, thickness and/or cross-sectional geometry.

In another aspect, the second portion of the suction valve may comprise a stop, which defines a maximum penetration depth of the second portion.

In other words, the first projection and/ or the second projection may comprise a stop. The stop may be in the form of a reinforcing rib or in the form of an annular rib or in the form of shoulders formed on the projection or the like.

Such a stop can simplify the assembly of the handle, reducing assembly time and thus assembly costs, since an assembler does not have to align the projection manually but simply pushes it in until the stop prevents further insertion.

In another aspect, the first half-shell may comprise retention hooks, which are formed directly adjacent to at least the first, outer fixing portion to close the housing.

In other words, latching geometries in the form of retention hooks may be formed between the first half-shell and the second half-shell to allow the handle to be closed or assembled without tools. In particular, the latching geometries may be formed directly adjacent to the first fixing portion to increase a clamping force on the first fixing portion and/ or the third fixing portion to improve sealing and fixation of the suction valve in the handle.

In another aspect, the valve body and/ or the stem are preferably made from a thermoplastic polymer, e.g. polypropylene (PP) especially biodegradable polypropylene, polystyrene (PS), polycarbonate (PC), acrylonitrile butadiene styrene (ABS), polyoxymethylene (POM), etc. However, also other polymer materials or other materials are conceivable.

The object of the present disclosure is further solved by a method of assembling an endoscope as described above, comprising the steps:
- Sliding a valve spring onto a valve body of a suction valve and fixing or latching the valve spring to a stem of the suction valve;
- Positioning the suction valve in a first half-shell of a housing of an endoscope handle or interface by inserting a second portion of the suction valve in a second, inner fixing portion of the first half-shell of the housing;
- Inserting a first portion of the suction valve, which is the valve spring, in a first, outer fixing portion of the first half-shell;
- Positioning a second half-shell of the housing on the first half-shell of the housing;
- Inserting the second portion of the suction valve in the fourth, inner fixing portion of the second half-shell of the housing;
- Inserting the first portion of the suction valve, which is the valve spring, in a third, outer fixing portion of the second half-shell;
- Closing the half-shells.

In other words, the object of the present disclosure is also solved by the method of assembling or mounting the endoscope according to any of the above aspects.

In a first step, the valve spring is fitted onto the valve body so that the valve body and valve spring are connected to each other. The connection between the valve spring and the valve body can be a force-fit and/or a form-fit connection.

In a second step, the suction valve is positioned in the first half-shell, with a first projection of the second portion of the valve body being inserted, preferably as far as the stop allows, into the second fixing portion.

In a third step, the first portion of the suction valve, which is the valve spring, is inserted into the first fixing portion of the first half-shell, which preferably comprises a groove.

In a fourth step, the two half-shells are positioned relative to each other so that their openings are facing each other.

In a fifth step, the two half-shells are joined together, with the second projection of the second portion of the suction valve being inserted into the fourth fixing portion. The fourth fixing portion preferably includes the V-shape to facilitate insertion.

In a sixth step, the first portion of the suction valve, which is the valve spring, is inserted into the third fixing portion of the second half-shell, which preferably comprises a groove.

In a seventh step, the first half-shell and the second half-shell are closed to form the handle of the endoscope. Preferably, latching hooks formed between the first half-shell and the second half-shell are engaged with each other. In other words, the first half-shell and the second half-shell are attached and/ or assembled to each other.

The present disclosure further relates to a system comprising the endoscope according to one of the above aspects and a video processing apparatus (VPA) couplable to the endoscope and capable of outputting a live image recorded by an image sensor of the endoscope on a display. The video processing apparatus may comprise a built-in display and/ or a detachable display and/or may be couplable to an external display. The video processing apparatus may be a video processing apparatus showing an image or a video captured by an image capturing means arranged at the distal tip unit.

Furthermore, the present disclosure relates to a system, that may also be claimed independently, comprising the endoscope described above wherein the system further comprises a suction device that is connectable directly or indirectly, for example through a hose, to the outlet of the valve body of the suction valve.

The suction device may be a vacuum pump or the like. There are also embodiments imaginable, in which the suction device is directly coupled to the endoscope or is part of the endoscope.

### Brief description of the figures

The disclosure is explained in more detail below using preferred embodiments and referring to the accompanying figures.
Fig. 1 shows a side view of an endoscope according to the present disclosure.
Fig. 2 shows a first half-shell of the endoscope handle or interface having a suction valve.
Fig. 3 shows the suction valve mounted in the first half-shell in an enlarged view.
Fig. 4 shows the first half-shell in a perspective view.
Fig. 5 shows a first enlarged view of the first half-shell.
Fig. 6 shows a second enlarged view of the first half-shell.
Fig. 7 shows a first enlarged view of a second half-shell.
Fig. 8 shows a second enlarged view of the second half-shell.
Fig. 9 shows a sectional view of the first half-shell and the second half-shell mounted to each other.
Fig. 10 shows a schematic view of the suction valve mounted in the handle.
Fig. 11 shows a sectional view of the suction valve mounted in the handle.
Fig. 12 shows a perspective view of the suction valve mounted in the first half-shell.
Fig. 13 shows a sectional view of the suction valve mounted between the first half-shell and the second half-shell.

The figures are schematic in nature and serve only to understand the disclosure. The features of the different embodiments can be interchanged among each other.

### Detailed description of the figures

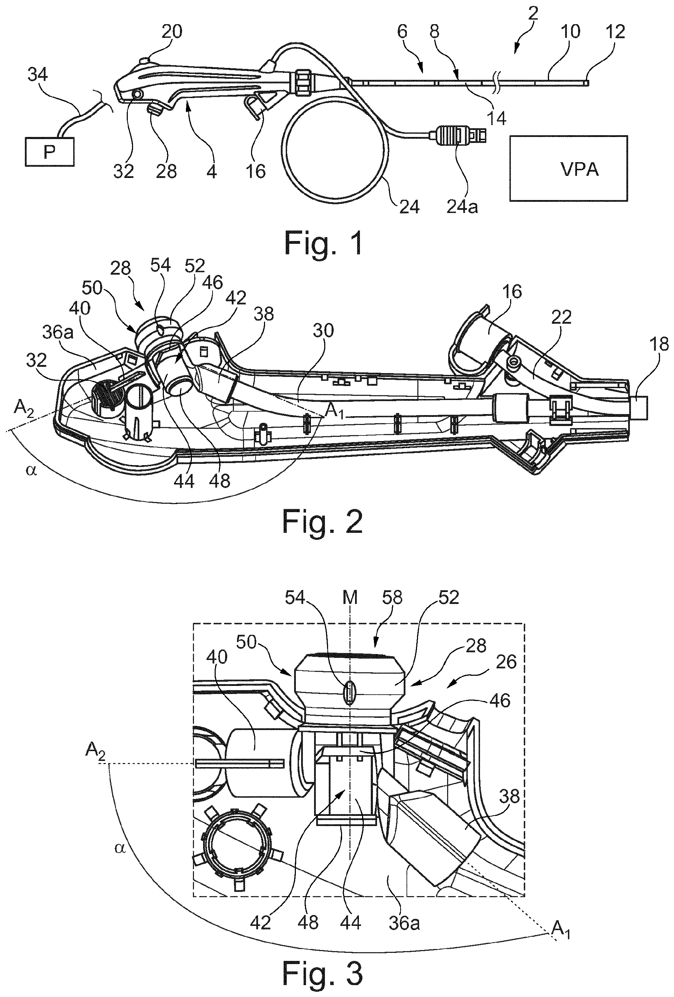

Fig. 1 shows a side view of an endoscope 2 according to the present disclosure, which is preferably a single-use endoscope. The endoscope 2 comprises: a proximal endoscope handle 4 and an insertion cord 6 extending distally from the endoscope handle 4. The insertion cord 6 is configured to be inserted into a patient's body cavity and comprises an insertion tube 8, a bending section 10 and a distal tip unit 12. The endoscope 2 further comprises a working channel 14 which extends from a biopsy port or working channel access port 16 provided at the endoscope handle 4 to the distal tip unit 12. The working channel 14 comprises a working channel tube 18 (shown in Fig. 2), which is arranged inside the endoscope handle 4, the insertion tube 8, and the bending section 10. The endoscope handle 4 comprises an operating unit 20, formed as a lever, for bending the bending section 10 of the insertion cord 6. The working channel tube 18 and the working channel access port 16 are connected via a Y-connector 22. The endoscope 2 is connectable to a video processing apparatus VPA, which is schematically illustrated via a connecting cable 24 comprising an electrical connector 24a insertable in a socket (not shown) of the video processing apparatus VPA. Alternatively the endoscope 2 may be wirelessly connectable to the video processing apparatus VPA, e.g. by a wireless HDMI connection.

When a surgical instrument or tool is inserted into the working channel 14, the surgical instrument or tool is inserted into the working channel access port 16 and guided through the Y-connector 22 into the working channel tube 18.

The endoscope 2 further comprises a suction valve 26, having a suction button 28 formed on the handle 4 and configured to control a suction power through the working channel 14 of the endoscope 2. The suction valve 26 is connected to a suction channel 30 and a vacuum port 32, which protrudes from or is embedded in the handle 4 (shown in Fig. 2). The endoscope 2 is connectable to a suction pump P, which is schematically illustrated in Fig. 1, via a hose 34, which is connectable to the vacuum port 32 of the endoscope 2.

Fig. 2 shows the handle 4 in an open state. Specifically, the handle 4 is formed essentially from two half-shells 36, a first half-shell 36a and a second half-shell 36b, that form the housing of the endoscope handle 4. In Fig. 2, the second half-shell 36b is removed and all other elements of the endoscope 2, which are not necessary for an understanding of the present disclosure, are also not shown in Fig. 2 for the sake of clarity and comprehensibility of the disclosure. It is to be understood that all other mechanical, optical and electronic components, which are formed in the handle 4 in a conventional endoscope, are or may be formed in the endoscope 2 of the present disclosure.

The suction channel 30 connects the suction valve 26, which is mounted between the first half-shell 36a and the second half-shell 36b at a proximal end portion of the handle 4, to the Y-connector 22 and runs in a longitudinal direction of the handle 4, that is, essentially in a direction, which continues the insertion cord 6. The suction valve 26 comprises an inlet 38, which is configured to connect to the suction channel 30. In other words, the inlet 38 is configured as a connector, which connects to the suction channel 30. The suction channel 30 has a tubular shape and may be in the form of an elastomeric tube or a rigid pipe. The suction valve 26 further comprises an outlet 40, which is connectable to the vacuum port 32. In other words, the outlet 40 is configured as a connector, which connects to the vacuum port 32.

In the following, the external structure of the suction valve 26 is explained in more detail with reference to Figs. 2 and 3. Fig. 3 is an enlarged view of the suction valve 26 in the assembled state, whereby the suction channel 30 and the vacuum port 32 are not shown in Fig. 3.

The suction valve 26 comprises a valve body 42, which comprises a valve cylinder 44 extending in an axial direction of the suction valve 26. The valve body 42 further comprises the inlet 38 and the outlet 40. The inlet 38 and the outlet 40 each have a substantially tubular geometry with a substantially circular cross-sectional configuration. Seen in the side view of the endoscope handle 4 in an assembled state of the suction valve in Fig. 3, the inlet 38 has a first center axis A1 and the outlet 40 has a second center axis A2. The first center axis A1 extends in a center position of the inlet 38 in a direction of extension of the inlet 38. The second center axis A2 extends in a center position of the outlet 40 in a direction of extension of the outlet 40. An angle α is enclosed between the first center axis A1 and the second center axis A2. The angle α is greater than 90° and less than 180°. Preferably, the angle is greater than 110°. Particularly preferably, the angle is about 120°.

The valve body 42 is formed in one piece from a plastic material, preferably polypropylene. Further, the valve body 42 comprises fixing projections 46 formed thereon, which are configured to fix the suction valve 26 in the handle 4 between the half-shells 36a and 36b. Specifically, the suction valve 26 includes two fixing projections 46 which extend oppositely radially outwardly away from the valve cylinder 44. The fixing projection 46 will be described in more detail below.

The suction valve 26 further includes an end cap 48 formed on an end portion of the valve cylinder 44 facing an interior of the handle 4, and closing the valve cylinder 44. Specifically, the end cap 48 closes a substantially circular bottom opening of the valve body 42. The end cap 48 and the valve body 42 are welded together. Alternatively, it is conceivable that the end cap 48 may be fixed in place by adhesive bonding or by a latching action.

A valve spring 50 is formed on an end portion of the valve cylinder 44 opposite the end cap 48. The valve spring 50 protrudes for the most part from the housing of the handle 4 and ensures that the suction valve 26 is maintained in an initial valve closed state or is returned to the initial valve closed state without a user actuation. A bypass hole 54 is formed in a side surface 52 of the valve spring 50. The bypass hole 54 allows ambient air to be drawn through the suction valve 26 into the outlet 40 in a closed state of the suction valve, that is, in a state in which the volume flow from inlet 38 to outlet 40 is blocked.

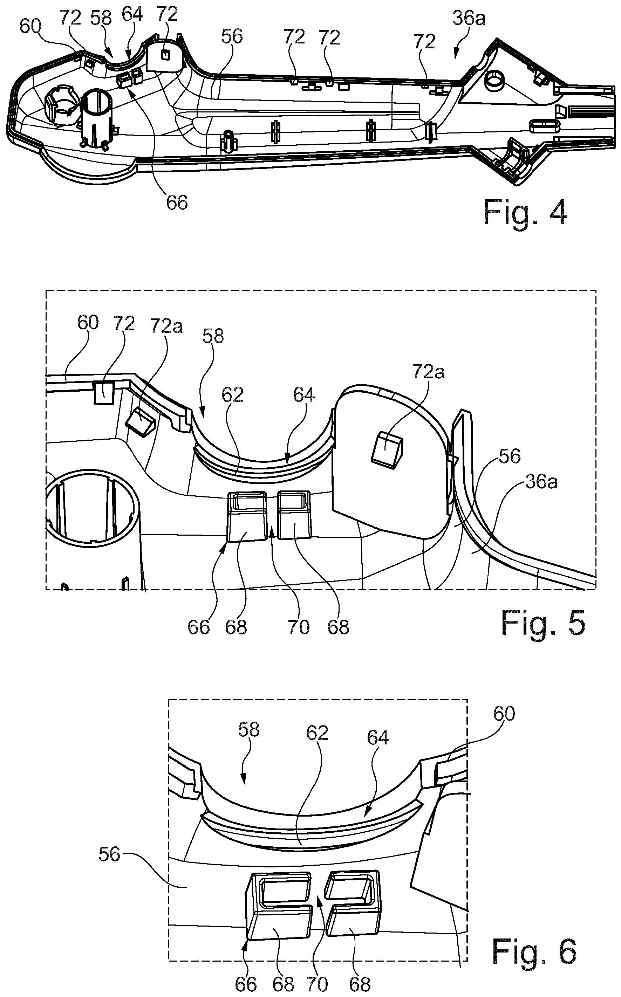

Fig. 4 shows the first half-shell 36a. Fig. 5 shows an enlarged view of the first half-shell 36a in an area of the first half-shell 36a in which the suction valve 26 is received. Fig. 6 shows a further enlarged view of receiving geometries in said area. In the following, the receiving geometries for the suction valve 26 in the first half-shell 36a are described in more detail with reference to Figs. 4, 5 and 6.

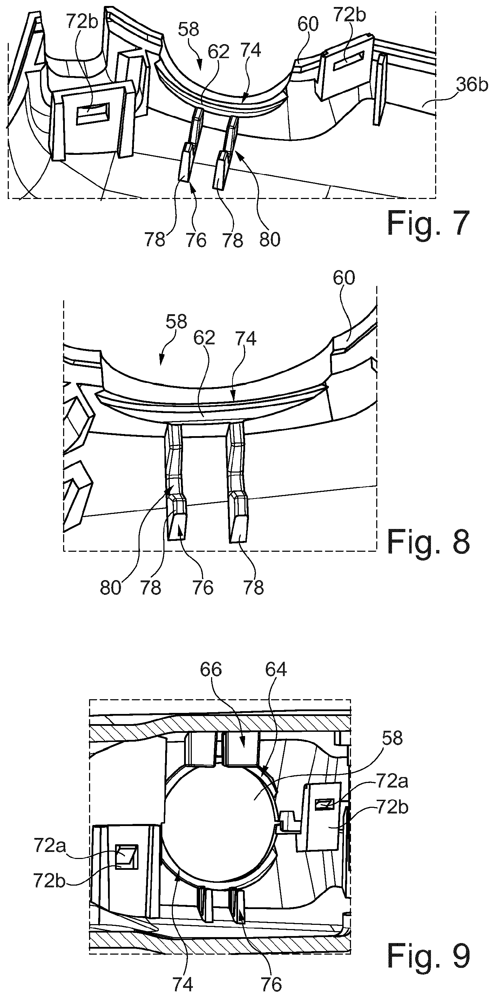

The first half-shell 36a comprises a shell skin 56 forming an outer wall of the handle 4. A recess 58 is formed in the shell skin 56, the recess 58 being configured to allow the suction valve 26 to extend outwardly from the interior of the handle 4 through the shell skin 56 of the handle 4. The recess 58 has a shape substantially in the form of a semicircle and extends from a contact edge 60 at which the first half-shell 36a and the second half-shell 36b abut one another in an assembled state of the handle 4. Specifically, the recess 58 of the first half-shell 36a is configured to form a circular opening in the shell skin 56 of the handle 4 in conjunction with the recess 58 of the second half-shell 36b when the first half-shell 36a and the second half-shell 36b are assembled (shown in Fig. 9).

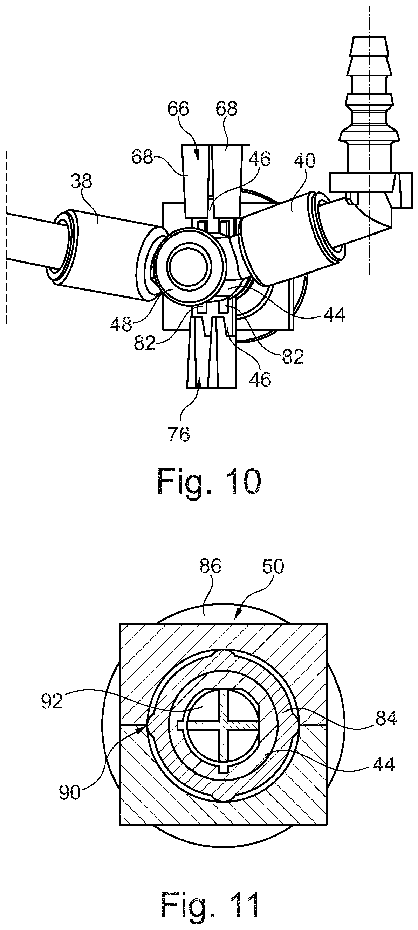

Directly adjacent to the recess 58, towards an interior space enclosed by the handle 4, a groove 62 is formed which, in conjunction with the recess 58, forms a first outer fixing portion 64 according to the preferred embodiment. The groove 62 extends into the shell skin 56, starting from the contact edge 60 in the area of the recess 58. The groove 62 may extend completely around the recess 58 or around a portion of the recess 58. The function of the first fixing portion 64 is described in more detail below with reference to Figures 10 to 13.

Parallel to the groove 62, offset towards the interior space of the handle 4, that is, further away from the recess 58, a second inner fixing portion 66 extends, starting from the shell skin 56, perpendicularly into the interior space. The second fixing portion 66 comprises two U-shaped brackets 68 extending away from the shell skin 56 facing each other and forming a substantially rectangular or slot shaped receptacle 70. The rectangular receptacle 70 is oriented parallel to the groove 62 and forms a cuboid-shaped receiving space. The two U-shaped brackets 68 face each other with arm sections of the U-shape. The two U-shaped brackets 68 extend from the shell skin 56 in a tower shape. The function of the second fixing portion 66 is described in more detail below with reference to Figures 10 to 13.

Embodiments are also conceivable, in which the receptacle 70 slightly tapers toward the shell skin 56.

Furthermore, embodiments are conceivable which are not formed from two U-shaped brackets 68 but from a single, preferably rectangular element having the rectangular or slot shaped receptacle 70.

The first half-shell 36a is a one-piece component injection molded from a plastic, preferably a thermoplastic.

Adjacent to the recess 58, retention hooks 72a are formed on the first half-shell 36a on the shell skin 56, which are configured to latch into corresponding counterparts 72b (shown in Fig. 7) in the second half-shell 36b to connect the first half-shell 36a and the second half-shell 36b together. Such retention hooks 72a are further evenly distributed around the entire circumference of the first half-shell 36a, preferably along the contact edge 60.

Fig.7 and Fig. 8 show the receiving geometries for the suction valve 26 in the second half-shell 36b. Since the second half-shell 36b corresponds in large parts to the first half-shell 36a, only differences between the second half-shell 36b and the first half-shell 36a will be described in the following.

Directly adjacent to the recess 58 of the second half-shell 36b, towards an interior space enclosed by the handle 4, the groove 62 is formed which, in conjunction with the recess 58, forms a third outer fixing portion 64. The groove 62 extends into the shell skin 56, starting from the contact edge 60 in the area of the recess 58.

Thus, the third fixing portion 74 of the second half-shell 36b corresponds to the first fixing portion 64 of the first half-shell 36a. In other words, the first fixing portion 64 and the third fixing portion 74 are identical in configuration. **In** still other words, the first fixing portion 64 and the third fixing portion 74 are symmetrical to each other with respect to a splitting layer or dividing plane of the handle 4. The splitting layer is a layer, which contains the contact edge 60.

Parallel to the groove 62, offset towards the interior space of the handle 4, that is, further away from the recess 58, a fourth inner fixing portion 76 extends, starting from the shell skin 56, perpendicularly into the interior space of the handle 4. The fourth fixing portion 76 comprises two V-shaped brackets 78 extending away from the shell skin 56 facing each other and forming a substantially V-shaped receptacle 80. The V-shaped receptacle 80 tapers from the interior space of the handle 4 to the shell skin 56. The V-shaped receptacle 80 is oriented parallel to the groove 62 and forms a three-sided straight prism-shaped receiving space. In other words, a receiving space that tapers toward the shell skin 56. The two V-shaped brackets 78 are each formed in a plate shape and parallel to each other. The two V-shaped brackets 78 extend from the shell skin 56 in a tower shape. The function of the second fixing portion 66 is described in more detail below with reference to Figures 10 to 13.

Furthermore, embodiments are conceivable which are not formed from two V-shaped brackets 78 but from a single, preferably rectangular element having the three-sided straight prism-shaped receiving space shaped receptacle 80.

In one embodiment, a portion of the V-shaped receptacle 80 that is formed closest to the shell skin 56 may include a parallel slot-shaped portion. In other words, the V-shaped receptacle 80 can change over its course into a regular rectangular slot shape.

The second half-shell 36b is a one-piece component injection molded from a plastic, preferably a thermoplastic.

Adjacent to the recess 58, the counterparts 72b are formed on the second half-shell 36b on the shell skin 56, which are configured to latch into the corresponding retention hooks 72a.

Fig. 9 shows the first half-shell 36a attached to the second half-shell 36b. The retention hooks 72a of the first half-shell 36a are mounted in the counterparts 72b of the second half-shell 36b. The recess 58 is configured as a circular opening in shell skin 56.

The first fixing portion 64 of the first half-shell 36a is positioned opposite the third fixing portion 74 of the second half-shell 36b. The second fixing portion 66 of the first half-shell 36a is positioned opposite the fourth fixing portion 76 of the second half-shell 36b.

Fig. 10 shows the suction valve 26 mounted in the first fixing portion 64, the second fixing portion 66, the third fixing portion 74 and the fourth fixing portion 76. A visualization of the half-shells 36 has been omitted for the sake of clarity. The fixing projections 46 are formed on the suction valve 26, which extend radially outward away from the valve cylinder 44. The fixing projections 46 are plate-shaped geometries, which are formed with supporting ribs 82. The ribs 82 also extend radially away from the valve cylinder 44 and extend over portions of the fixing projection 46. Two ribs 82 are formed on each of the fixing projections 46.

In the assembled state, a first of the fixing projections 46 is accommodated in the second fixing portion 66. In other words, the first of the fixing projections 46 is inserted into the second fixing portion 66 in such a way that the two U-shaped brackets 68 circumferentially or peripherally surround the first of the fixing projections 46 and the first of the fixing projections 46 is in the cuboid-shaped receiving space.

The ribs 82 provided on the first of the fixing projections 46 form a stop. In other words, the first of the fixing projections 46 can only be inserted into the second fixing portion 66 until the ribs 82 abut the second fixing portion 66.

A second of the fixing projections 46 is inserted in the fourth fixing portion 76. In other words, the second of the fixing projections 46 is inserted into the fourth fixing portion 76 in such a way that the two V-shaped brackets 68 accommodate the second of the fixing projections.

Further, the suction valve 26 is mounted to the first fixing portion 64 and the third fixing portion 74. Specifically, the valve spring 50 of the suction valve 26 is connected to the housing of the handle 4 via the first fixing portion 64 and the third fixing portion 74.

The fixation of the valve spring 50 is shown in Fig. 11 and will be explained in more detail below.

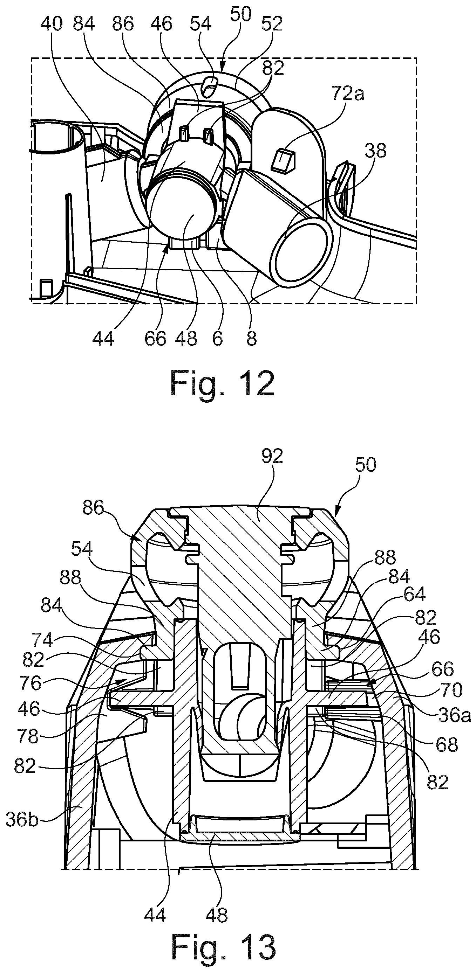

The valve spring 50 comprises a collar or flange 84 and a valve spring main body 86. The collar 84 is fixed by the first fixing portion 64 and the third fixing portion 74. The collar 84 and the valve spring main body 86 are connected to each other by a neck 88 (shown in Fig. 13). The first fixing portion 64 and the third fixing portion 74 surround the valve spring 50 in the area of the neck 88 in such a way that they fully surround the valve spring 50 in the area of the neck 88. The collar 84 is thereby positioned in the groove 62. The collar 84 comprises four tolerance absorbing bumps 90, which are each offset by 90° around the circumference of the collar 84.

In the following, the assembly of the suction valve 26 in the handle 4 is explained in more detail with reference to Figs. 12 and 13.

In a first step, shown in Fig. 12, the suction valve 26 is positioned in the first half-shell 36a. For this purpose, the first of the fixing projections 46 is inserted into the second fixing portion 66 and the neck 88 of the valve spring 50 is positioned in the first fixing portion 64. Specifically, the first of the fixing projections 46 is slid between the two U-shaped brackets 68 until the ribs 82 rest against the U-shaped brackets 68. Further, the collar 84 is positioned in the groove 62.

In a second step shown in Fig. 13, the first half-shell 36a and the second half-shell 36b are put together. Therefore, the second of the fixing projections 46 is inserted into the fourth fixing portion 76. The V-shape of the fourth fixing portion 76 makes insertion of the second of the fixing projections 46 into the fourth fixing portion 76 much easier. Furthermore, the neck 88 of the valve spring 50 is positioned in the third fixing portion 74. Therefore, the collar 84 is positioned in the groove 62. Subsequently, the handle 4 is assembled from the first half-shell 36a and the second half-shell 36b by snapping the retention hooks 72a into the counterparts 72b.

Of course, other electrical and mechanical components and assemblies may be positioned in the handle 4 prior to assembling the first half-shell 36a and the second half-shell 36b.

### List of reference numbers

- 2: endoscope
- 4: endoscope handle
- 6: insertion cord
- 8: insertion tube
- 10: bending section
- 12: distal tip unit
- 14: working channel
- 16: working channel access port
- 18: working channel tube
- 20: operating unit
- 22: Y-connector
- 24: connecting cable
- 24a: electrical connector
- 26: suction valve
- 28: suction button
- 30: suction channel
- 32: vacuum port
- 34: hose
- 36: half-shell
- 36a: first half-shell
- 36b: second half-shell
- 38: inlet
- 40: outlet
- 42: valve body
- 44: valve cylinder
- 46: fixing projections
- 48: end cap
- 50: valve spring
- 52: side surface
- 54: bypass hole
- 56: shell skin
- 58: recess
- 60: contact edge
- 62: groove
- 64: first fixing portion
- 66: second fixing portion
- 68: U-shaped bracket
- 70: slot shaped receptacle
- 72a: retention hook
- 72b: counterpart
- 74: third fixing portion
- 76: fourth fixing portion
- 78: V-shaped bracket
- 80: V-shaped receptacle
- 82: rib
- 84: collar/ flange
- 86: spring main body
- 88: neck
- 90: bump
- 92: stem
- A1: first center axis
- A2: second center axis
- α: angle
- P: suction pump
- VPA: video processing apparatus

## Claims

1. An endoscope (2) comprising:
a proximal endoscope handle or interface (4);
an insertion cord (6) extending from the endoscope handle or interface (4) and configured to be inserted into a patient's body cavity;
a working channel (14) configured to let fluid and mucus sucked from the patient's body cavity flow therethrough;
the endoscope handle or interface (4) comprising:
a housing; and
a suction valve (26) configured to control a suction through the working channel (14) and having a valve closed state and a valve open state;
the housing comprising a first half-shell (36a) and a second half-shell (36b);
the suction valve (26) comprising:
a valve body (42) having:
an inlet connected (38) to the working channel (14);
an outlet (40) provided and configured to be connected to a suction device (P); and
a stem (92) movable axially in the valve body (42) between at least a first position, in which the suction valve (26) is in the valve closed state, and a second position, in which the suction valve is in the valve open state;
wherein the suction valve (26) is mounted between the first half-shell (36a) and the second half-shell (36b); and wherein
the first half-shell (36a) comprises
a first, outer fixing portion (64) and
a second, inner fixing (66) portion,
wherein the first, outer fixing portion (64) is configured to receive a first portion of the suction valve (26), which is a valve spring (50), and wherein the second, inner fixing portion (66) is configured to receive a second portion of the suction valve (26),
wherein the second half-shell (36b) comprises
a third, outer fixing portion (74) and
a fourth, inner fixing portion (76),
wherein the third, outer fixing portion (74) is configured to receive the first portion of the suction valve (26), and wherein the fourth, inner fixing portion (76) is configured to receive the second portion of the suction valve (26),
wherein the valve spring (50) is formed by an elastic material, and the first outer fixing portion (64) of the first half-shell (36a) and the third outer fixing portion (74) of the second half-shell (36b) contact and clamp the valve spring (50) of the suction valve (26) in an assembled state of the first and second half-shells (36).

2. The endoscope (2) according to claim 1, wherein the first, outer fixing portion (64) of the first half-shell (36a) and the third, outer fixing portion (74) of the second half-shell (36b) are formed identically or symmetrically to each other.

3. The endoscope (2) according to claim 2, wherein the first, outer fixing portion (64) of the first half-shell (36a) and the third, outer fixing portion (74) of the second half-shell (36b) encircle the first portion of the suction valve (26) in a circumferential direction.

4. The endoscope (2) according to one of claims 1 to 3, wherein the second, inner fixing portion (66) of the first half-shell (36a) and the fourth inner fixing portion (76) of the second half-shell (36b) have a different geometry.

5. The endoscope (2) according to claim 4, wherein the second, inner fixing portion (66) of the first half-shell (36a) comprises a slot (70) of constant width and the fourth inner fixing portion (76) of the second half-shell (36b) comprises a V-shaped slot (80).

6. The endoscope (2) according to one of claims 1 to 5, wherein the second, inner fixing portion (66) of the first half-shell (36a) and the fourth inner fixing portion (76) of the second half-shell (36b) are configured as tower geometries protruding from the respective half-shells (36a; 36b) into an interior space of the endoscope handle or interface (4).

7. The endoscope (2) according to one of claims 1 to 6, wherein the second portion of the suction valve (26) is or comprises at least one projection (46) formed integrally with the valve body (42).

8. The endoscope (2) according to claim 7, wherein the second portion of the suction valve (26) comprises
a first projection (46) formed integrally with the valve body (42) and
a second projection (46) formed integrally with the valve body (42),
said first projection (46) and said second projection (46) projecting in essentially opposite directions from said valve body (42).

9. The endoscope (2) according to claim 7 or 8, wherein the second portion of the suction valve (26) comprises a stop (82), which defines a maximum penetration depth of the second portion.

10. The endoscope (2) according to one of claims 1 to 9, wherein the first half-shell (36a) comprises retention hooks (72a), which are formed directly adjacent to at least the first, outer fixing portion (64) to close the housing of the endoscope handle or interface (4).

11. A method of assembling an endoscope (2) according to any of claims 1 to 10, comprising the steps:
- Sliding the valve spring (50) onto the valve body (42) of the suction valve (26) and latching the valve spring (50) to the stem (92) of the suction valve (26);
- Positioning the suction valve (26) in the first half-shell (36a) of the housing of an endoscope handle or interface (4) by inserting the second portion of the suction valve (26) in the second, inner fixing portion (66) of the first half-shell (36a) of the housing;
- Inserting the first portion of the suction valve (26), which is the valve spring (50), in the first, outer fixing portion (64) of the first half-shell (36a);
- Positioning the second half-shell (36b) of the housing on the first half-shell (36a) of the housing;
- Inserting the second portion of the suction valve (26) in the fourth, inner fixing portion (76) of the second half-shell (36b) of the housing;
- Inserting the first portion of the suction valve (26) in the third, outer fixing portion (74) of the second half-shell (36b);
- Closing the half-shells (36a; 36b).

12. A system comprising an endoscope (2) according to one of claims 1 to 10 and a video processing apparatus (VPA).

## Patentansprüche

1. Endoskop (2), mit:
einem proximalen Endoskopgriff oder Endoskopschnittstelle (4);
einem Einführstrang (6), der sich von dem Endoskopgriff oder der Endoskopschnittstelle (4) erstreckt und dazu ausgebildet ist, in den Körperhohlraum eines Patienten eingeführt zu werden;
einem Arbeitskanal (14), der dazu ausgebildet ist, Fluid und Schleim, die aus dem Körperhohlraum des Patienten angesaugt werden, hindurchströmen zu lassen;
wobei der Endoskopgriff oder die Endoskopschnittstelle (4) Folgendes aufweist:
ein Gehäuse; und
ein Saugventil (26), das dazu ausgebildet ist, eine Saugwirkung durch den Arbeitskanal (14) zu steuern, und das einen geschlossenen Ventilzustand und einen offenen Ventilzustand aufweist;
wobei das Gehäuse eine erste Halbschale (36a) und eine zweite Halbschale (36b) aufweist;
wobei das Saugventil (26) Folgendes aufweist:
einen Ventilkörper (42), der Folgendes hat:
einen Einlass (38), der mit dem Arbeitskanal (14) verbunden ist;
einen Auslass (40), der vorgesehen und dazu ausgebildet ist, mit einer Saugvorrichtung (P) verbunden zu werden; und
einen Schaft (92), der in dem Ventilkörper (42) zwischen wenigstens einer ersten Position, in der sich das Saugventil (26) in dem geschlossenen Ventilzustand befindet, und einer zweiten Position, in der sich das Saugventil in dem offenen Ventilzustand befindet, axial bewegbar ist;
wobei das Saugventil (26) zwischen der ersten Halbschale (36a) und der zweiten Halbschale (36b) montiert ist; und wobei
die erste Halbschale (36a) Folgendes aufweist:
einen ersten, äußeren Befestigungsabschnitt (64) und
einen zweiten, inneren Befestigungsabschnitt (66),
wobei der erste, äußere Befestigungsabschnitt (64) dazu ausgebildet ist, einen ersten Abschnitt des Saugventils (26) aufzunehmen, der eine Ventilfeder (50) ist, und wobei der zweite, innere Befestigungsabschnitt (66) dazu ausgebildet ist, einen zweiten Abschnitt des Saugventils (26) aufzunehmen,
wobei die zweite Halbschale (36b) Folgendes aufweist:
einen dritten, äußeren Befestigungsabschnitt (74) und
einen vierten, inneren Befestigungsabschnitt (76),
wobei der dritte, äußere Befestigungsabschnitt (74) dazu ausgebildet ist, den ersten Abschnitt des Saugventils (26) aufzunehmen, und wobei der vierte, innere Befestigungsabschnitt (76) dazu ausgebildet ist, den zweiten Abschnitt des Saugventils (26) aufzunehmen,
wobei die Ventilfeder (50) durch ein elastisches Material gebildet ist und der erste äußere Befestigungsabschnitt (64) der ersten Halbschale (36a) und der dritte äußere Befestigungsabschnitt (74) der zweiten Halbschale (36b) die Ventilfeder (50) des Saugventils (26) in einem zusammengebauten Zustand der ersten und der zweiten Halbschale (36) berühren und festklemmen.

2. Endoskop (2) nach Anspruch 1, wobei der erste, äußere Befestigungsabschnitt (64) der ersten Halbschale (36a) und der dritte, äußere Befestigungsabschnitt (74) der zweiten Halbschale (36b) identisch oder symmetrisch zueinander ausgebildet sind.

3. Endoskop (2) nach Anspruch 2, wobei der erste, äußere Befestigungsabschnitt (64) der ersten Halbschale (36a) und der dritte, äußere Befestigungsabschnitt (74) der zweiten Halbschale (36b) den ersten Abschnitt des Saugventils (26) in einer Umfangsrichtung umgeben.

4. Endoskop (2) nach einem der Ansprüche 1 bis 3, wobei der zweite, innere Befestigungsabschnitt (66) der ersten Halbschale (36a) und der vierte, innere Befestigungsabschnitt (76) der zweiten Halbschale (36b) eine unterschiedliche Geometrie aufweisen.

5. Endoskop (2) nach Anspruch 4, wobei der zweite, innere Befestigungsabschnitt (66) der ersten Halbschale (36a) einen Schlitz (70) mit konstanter Breite aufweist und der vierte, innere Befestigungsabschnitt (76) der zweiten Halbschale (36b) einen V-förmigen Schlitz (80) aufweist.

6. Endoskop (2) nach einem der Ansprüche 1 bis 5, wobei der zweite, innere Befestigungsabschnitt (66) der ersten Halbschale (36a) und der vierte, innere Befestigungsabschnitt (76) der zweiten Halbschale (36b) als Turmgeometrien ausgebildet sind, die von den jeweiligen Halbschalen (36a; 36b) in einen Innenraum des Endoskopgriffs oder der Endoskopschnittstelle (4) vorstehen.

7. Endoskop (2) nach einem der Ansprüche 1 bis 6, wobei der zweite Abschnitt des Saugventils (26) mindestens ein Vorsprung (46) ist oder aufweist, der integral mit dem Ventilkörper (42) ausgebildet ist.

8. Endoskop (2) nach Anspruch 7, wobei der zweite Abschnitt des Saugventils (26) Folgendes aufweist:
einen ersten Vorsprung (46), der integral mit dem Ventilkörper (42) ausgebildet ist, und
einen zweiten Vorsprung (46), der integral mit dem Ventilkörper (42) ausgebildet ist,
wobei der erste Vorsprung (46) und der zweite Vorsprung (46) im Wesentlichen in entgegengesetzte Richtungen von dem Ventilkörper (42) vorstehen.

9. Endoskop (2) nach Anspruch 7 oder 8, wobei der zweite Abschnitt des Saugventils (26) einen Anschlag (82) aufweist, der eine maximale Eindringtiefe des zweiten Abschnitts definiert.

10. Endoskop (2) nach einem der Ansprüche 1 bis 9, wobei die erste Halbschale (36a) Haltehaken (72a) aufweist, die direkt benachbart zu zumindest dem ersten, äußeren Befestigungsabschnitt (64) ausgebildet sind, um das Gehäuse des Endoskopgriffs oder der Endoskopschnittstelle (4) zu schließen.

11. Verfahren zum Zusammenbauen eines Endoskops (2) nach einem der Ansprüche 1 bis 10, mit den Schritten:
- Schieben der Ventilfeder (50) auf den Ventilkörper (42) des Saugventils (26) und Verriegeln der Ventilfeder (50) an dem Schaft (92) des Saugventils (26);
- Positionieren des Saugventils (26) in der ersten Halbschale (36a) des Gehäuses eines Endoskopgriffs oder einer Endoskopschnittstelle (4) durch Einführen des zweiten Abschnitts des Saugventils (26) in den zweiten, inneren Befestigungsabschnitt (66) der ersten Halbschale (36a) des Gehäuses;
- Einführen des ersten Abschnitts des Saugventils (26), der die Ventilfeder (50) ist, in den ersten, äußeren Befestigungsabschnitt (64) der ersten Halbschale (36a);
- Positionieren der zweiten Halbschale (36b) des Gehäuses an der ersten Halbschale (36a) des Gehäuses;
- Einführen des zweiten Abschnitts des Saugventils (26) in den vierten, inneren Befestigungsabschnitt (76) der zweiten Halbschale (36b) des Gehäuses;
- Einführen des ersten Abschnitts des Saugventils (26) in den dritten, äußeren Befestigungsabschnitt (74) der zweiten Halbschale (36b);
- Schließen der Halbschalen (36a; 36b).

12. System mit einem Endoskop (2) nach einem der Ansprüche 1 bis 10 und einer Videoverarbeitungsvorrichtung (VPA).

## Revendications

1. Endoscope (2), comprenant :
une poignée ou une interface d'endoscope proximale (4) ;
un cordon d'insertion (6) s'étendant de la poignée ou de l'interface d'endoscope (4) et configuré pour être inséré dans une cavité corporelle d'un patient ;
un canal de travail (14) configuré pour laisser du fluide et/ou du mucus aspiré de la cavité corporelle du patient s'écouler par celui-ci ;
la poignée ou l'interface d'endoscope (4) comprenant :
un logement ; et
une soupape d'aspiration (26) configurée pour commander une aspiration à travers le canal de travail (14) et présentant un état fermé de soupape et un état ouvert de soupape ;
le logement comprenant une première demi-coque (36a) et une seconde demi-coque (36b) ;
la soupape d'aspiration (26) comprenant :
un corps de soupape (42) présentant :
une entrée (38) connectée au canal de travail (14) ;
une sortie (40) prévue et configurée pour être connectée à un dispositif d'aspiration (P) ; et
une tige (92) mobile axialement dans le corps de soupape (42) entre au moins une première position dans laquelle la soupape d'aspiration (26) est dans l'état fermé de soupape, et une seconde position dans laquelle la soupape d'aspiration est dans l'état ouvert de soupape ;
dans lequel la soupape d'aspiration (26) est montée entre la première demi-coque (36a) et la seconde demi-coque (36b) ; et dans lequel
la première demi-coque (36a) comprend
une première partie de fixation extérieure (64) et
une deuxième partie de fixation intérieure (66),
dans lequel la première partie de fixation extérieure (64) est configurée pour recevoir une première partie de la soupape d'aspiration (26), qui est un ressort de soupape (50), et dans lequel la seconde partie de fixation intérieure (66) est configurée pour recevoir une seconde partie de la soupape d'aspiration (26),
dans lequel la seconde demi-coque (36b) comprend
une troisième partie de fixation extérieure (74) et
une quatrième partie de fixation intérieure (76),
dans lequel la troisième partie de fixation extérieure (74) est configurée pour recevoir la première partie de la soupape d'aspiration (26), et dans lequel la quatrième partie de fixation intérieure (76) est configurée pour recevoir la deuxième partie de la soupape d'aspiration (26),
dans lequel le ressort de soupape (50) est formé d'un matériau élastique, et la première partie de fixation extérieure (64) de la première demi-coque (36a) et la troisième partie de fixation extérieure (74) de la deuxième demi-coque (36b) viennent en contact et serrent le ressort de soupape (50) de la soupape d'aspiration (26) dans un état assemblé des première et seconde demi-coques (36).

2. Endoscope (2) selon la revendication 1, dans lequel la première partie de fixation extérieure (64) de la première demi-coque (36a) et la troisième partie de fixation extérieure (74) de la seconde demi-coque (36b) sont formées de manière identique ou symétrique l'une par rapport à l'autre.

3. Endoscope (2) selon la revendication 2, dans lequel la première partie de fixation extérieure (64) de la première demi-coque (36a) et la troisième partie de fixation extérieure (74) de la seconde demi-coque (36b) entourent la première partie de la soupape d'aspiration (26) dans une direction circonférentielle.

4. Endoscope (2) selon l'une des revendications 1 à 3, dans lequel la deuxième partie de fixation intérieure (66) de la première demi-coque (36a) et la quatrième partie de fixation intérieure (76) de la seconde demi-coque (36b) présentent une géométrie différente.

5. Endoscope (2) selon la revendication 4, dans lequel la deuxième partie de fixation intérieure (66) de la première demi-coque (36a) comprend une fente (70) de largeur constante et la quatrième partie de fixation intérieure (76) de la seconde demi-coque (36b) comprend une fente en forme de V (80).

6. Endoscope (2) selon l'une des revendications 1 à 5, dans lequel la deuxième partie de fixation intérieure (66) de la première demi-coque (36a) et la quatrième partie de fixation intérieure (76) de la seconde demi-coque (36b) sont configurées comme des géométries de tour faisant saillie à partir des demi-coques respectives (36a ; 36b) dans un espace intérieur de la poignée ou de l'interface d'endoscope (4).

7. Endoscope (2) selon l'une des revendications 1 à 6, dans lequel la seconde partie de la soupape d'aspiration (26) est ou comprend au moins une saillie (46) formée d'un seul tenant avec le corps de soupape (42).

8. Endoscope (2) selon la revendication 7, dans lequel la seconde partie de la soupape d'aspiration (26) comprend
une première saillie (46) formée d'un seul tenant avec le corps de soupape (42) et
une seconde saillie (46) formée d'un seul tenant avec le corps de soupape (42),
ladite première saillie (46) et ladite seconde saillie (46) faisant saillie dans des directions essentiellement opposées à partir dudit corps de soupape (42).

9. Endoscope (2) selon la revendication 7 ou 8, dans lequel la seconde partie de la soupape d'aspiration (26) comprend une butée (82), qui définit une profondeur de pénétration maximale de la seconde partie.

10. Endoscope (2) selon l'une des revendications 1 à 9, dans lequel la première demi-coque (36a) comprend des crochets de retenue (72a), qui sont formés directement à proximité d'au moins la première partie de fixation extérieure (64) pour fermer le logement de la poignée ou de l'interface d'endoscope (4).

11. Procédé d'assemblage d'un endoscope (2) selon l'une quelconque des revendications 1 à 10, comprenant les étapes :
- faire coulisser le ressort de soupape (50) sur le corps de soupape (42) de la soupape d'aspiration (26) et verrouiller le ressort de soupape (50) sur la tige (92) de la soupape d'aspiration (26) ;
- positionner la soupape d'aspiration (26) dans la première demi-coque (36a) du logement d'une poignée ou d'une interface d'endoscope (4) en insérant la deuxième partie de la valve d'aspiration (26) dans la deuxième partie de fixation intérieure (66) de la première demi-coque (36a) du logement ;
- insérer la première partie de la soupape d'aspiration (26), qui est le ressort de soupape (50), dans la première partie de fixation extérieure (64) de la première demi-coque (36a) ;
- positionner la seconde demi-coque (36b) du logement sur la première demi-coque (36a) du logement ;
- insérer la seconde partie de la soupape d'aspiration (26) dans la quatrième partie de fixation intérieure (76) de la seconde demi-coque (36b) du logement ;
- insérer la première partie de la soupape d'aspiration (26) dans la troisième partie de fixation extérieure (74) de la seconde demi-coque (36b) ;
- fermer les demi-coques (36a ; 36b).

12. Système comprenant un endoscope (2) selon l'une des revendications 1 à 10 et un appareil de traitement vidéo (VPA).
